# EUROPEAN PATENT SPECIFICATION

(11) **EP 2 464 616 B1**
(45) Date of publication and mention of the grant of the patent: **29.10.2014**
(21) Application number: 10805566.6
(22) Date of filing: 04.08.2010
(51) Int. Cl.: C07C 13/62, C07C 15/20, C07D 209/86, C07D 209/82, C07D 213/06, C07D 217/02, C09K 11/06, H01L 51/50

(54) **ORGANIC COMPOUND AND ORGANIC LIGHT-EMITTING DEVICE USING THE SAME**
ORGANISCHE VERBINDUNG UND ORGANISCHE LICHTEMITTIERENDE VORRICHTUNG DAMIT
COMPOSÉ ORGANIQUE ET DISPOSITIF ORGANIQUE ÉMETTEUR DE LUMIÈRE L'EMPLOYANT

(30) Priority: 10.08.2009 JP 2009185556
(43) Date of publication of application: 20.06.2012
(73) Proprietor: Canon Kabushiki Kaisha, Tokyo, 1468501 (JP)
(72) Inventor: KAMATANI, Jun, Tokyo 1468501 (JP); HORIUCHI, Takayuki, Tokyo 1468501 (JP); YAMADA, Naoki, Tokyo 1468501 (JP); SAITOH, Akihito, Tokyo 1468501 (JP)
(74) Representative: TBK
(86) International application number: PCT/JP2010/004896
(87) International publication number: WO 2011/018886

(56) References cited:
- EP-A1- 2 085 371
- WO-A1-2008/015945
- WO-A1-2008/059713
- WO-A1-2010/018842
- WO-A1-2010/018842
- JP-A- 10 189 247
- JP-A- 2003 347 058
- JP-A- 2005 235 787
- JP-A- 2010 143 880
- JP-A- 2010 147 332
- US-A1- 2004 076 853
- YIANNIS A. LEVENDIS ET AL.: 'Emissions from burning tire-derived fuel (TDF) : comparison of batch combustion of tire chips and continuous combustion of tire crumb mixed with coal' THE PROCEEDINGS OF THE 23RD INTERNATIONAL TECHNICAL CONFERENCE ON COAL UTILIZATION & FUEL SYSTEMS 1998, pages 95 - 106, XP008152839

## Description

### Technical Field

The present invention relates to an organic compound that has good emission characteristics and an organic light-emitting device using the organic compound.

### Background Art

Organic light-emitting devices (also known as organic electroluminescent devices or organic EL devices) are a type of light-emitting device that includes a thin film containing a fluorescent organic compound interposed between an anode and a cathode. When electrons and holes are injected from the respective electrodes, excitons of the fluorescent compound are generated and the organic light-emitting device emits light as the excitons return to their ground state.

The recent advancement of organic light-emitting devices has been remarkable. Organic light-emitting devices make it possible to produce thin and light-weight light-emitting devices that have high luminance at a low application voltage and a wide variety of emission wavelengths and display rapid response. This suggests that the organic light-emitting devices can be used in a wide variety of usages.

In order to provide organic light-emitting devices that have ever higher performance, development of constituent materials of organic light-emitting devices has been pursued as actively as organic light-emitting devices. Various compounds have been proposed so far.

For example, PTL 1 to PTL 4 propose organic compounds that can be used as the constituent material of emission layers.

US 2004076853 (A1) describes an organic light-emitting device including a substrate, an anode and a cathode disposed over the substrate, and a luminescent layer disposed between the anode and the cathode wherein the luminescent layer includes a host and at least one dopant. The host of the luminescent layer is selected to include a solid organic material comprising a mixture of at least two components, one of which is capable of forming both monomer state and an aggregate state. The first component of the mixture may be a benzenoid compound that has the formula: 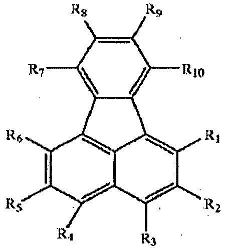 wherein substituents R1 through R10 are each individually hydrogen, fluoro, cyano, alkoxy, aryloxy, diarylamino, akylalkyamino, dialkylamino, trialkylsilyl, triarylsilyl, diarylalkylsilyl, dialkylarysilyl, keto, dicyanomethyl, alkyl of from 1 to 24 carbon atoms, alkenyl of from 1 to 24 carbon atoms, alkynyl of from 1 to 24 carbon atoms, aryl of from 5 to 30 carbon atoms, substituted aryl, heterocycle containing at least one nitrogen atom, or at least one oxygen atom, or at least one sulfur atom, or at least one boron atom, or at least one phosphorus atom, or at least one silicon atom, or any combination thereof; or any two adjacent R1 through R10 substituents form an annelated benzo-, naphtho-, anthra-, phenanthro-, fluorantheno-, pyreno-, triphenyleno-, or peryleno-substituent or its alkyl or aryl substituted derivative; or any two R1 through R10 substituents form a 1,2-benzo, 1,2-naphtho, 2,3-naphtho, 1,8-naphtho, 1,2-anthraceno, 2,3-anthraceno, 2,2'-BP, 4,5-PhAn, 1,12-TriP, 1,12-Per, 9,10-PhAn, 1,9-An, 1,10-PhAn, 2,3-PhAn, 1,2-PhAn, 1,10-Pyr, 1,2-Pyr, 2,3-Per, 3,4-FlAn, 2,3-FlAn, 1,2-FlAn, 3,4-Per, 7,8-FlAn, 8,9-FlAn, 2,3-TriP, 1,2-TriP, ace, or indeno substituent or their alkyl or aryl substituted derivative.

WO 2010018842 (A1) (Art. 54(3) EPC) describes a benzofluoranthene derivative represented by formula (1): 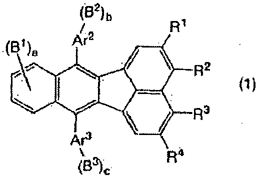 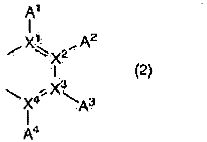 wherein at least one pair of "R1 and R2" and "R3 and R4" are linked to each other to form a ring represented by formula (2).

### Citation List

### Patent Literature

PTL 1: Japanese Patent No. 3853042
PTL 2: Japanese Patent No. 4002277
PTL 3: WO2008-015945
PTL 4: WO2008-059713

### Non Patent Literature

NPL 1: Journal of Organic Chemistry, 17, 845-54 (1952)

### Summary of Invention

Organic compounds and organic light-emitting devices described in PTL 1 to PTL 4 need some improvements before they can be sufficiently used in practical applications.

To be more specific, emitted light needs to have a higher luminance and the optical conversion efficiency needs to be increased for practical applications. Moreover, improvements are needed in terms of durability, such as changes with time caused by long use and deterioration caused by oxygen-containing atmospheric gas and humidity. In order for organic light-emitting devices to be used in full color displays and the like, the color purity must be high and the blue light must be emitted at a high efficiency. However, these problems have not been sufficiently addressed. Accordingly, organic light-emitting devices that exhibit high color purity, emission efficiency, and durability and the materials that can be used to make such devices are in demand.

An organic compound represented by general formula (1) below is provided:

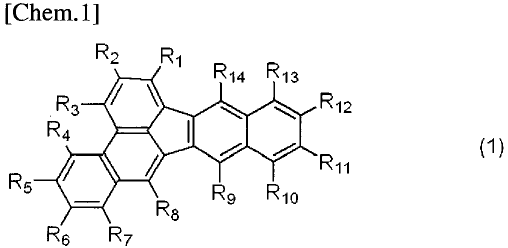

In formula (1), R₁ to R₁₄ each independently represent a hydrogen atom, a halogen atom, a substituted or unsubstituted alkyl group, a substituted or unsubstituted alkoxy group, a substituted or unsubstituted amino group, a substituted or unsubstituted aryl group, or a substituted or unsubstituted heterocyclic group, and R₁₁ or R₁₂ represents a substituted or unsubstituted fused polycyclic aromatic group.

### Brief Description of Drawings

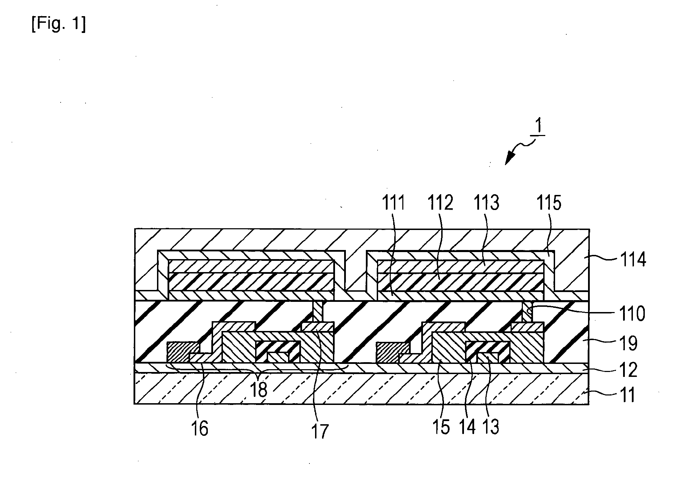

[fig. 1]Fig. 1 is a cross-sectional view of an example of an image display apparatus equipped with an organic light-emitting device according to one embodiment of the present invention.

### Description of Embodiments

An organic compound according to an embodiment of the present invention is first described. The organic compound is represented by general formula (1) below.

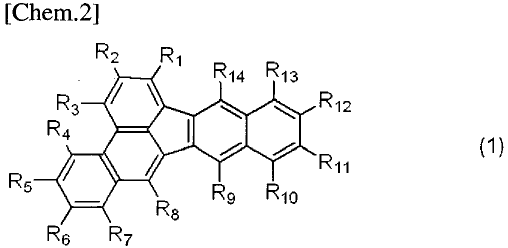

In formula (1), R₁ to R₁₄ each independently represent a hydrogen atom, a halogen atom, a substituted or unsubstituted alkyl group, a substituted or unsubstituted alkoxy group, a substituted or unsubstituted amino group, a substituted or unsubstituted aryl group, or a substituted or unsubstituted heterocyclic group.

Examples of the halogen atom represented by R₁ to R₁₄ include, but are not limited to, fluorine, chlorine, bromine, and iodine atoms.

Examples of the alkyl groups represented by R₁ to R₁₄ include, but are not limited to, a methyl group, an ethyl group, a normal propyl group, an isopropyl group, a normal butyl group, a tertiary butyl group, a secondary butyl group, an octyl group, a 1-adamantyl group, and a 2-adamantyl group.

Examples of the alkoxy group represented by R₁ to R₁₄ include, but are not limited to, a methoxy group, an ethoxy group, a propoxy group, a 2-ethyl-octyloxy group, and a benzyloxy group.

Examples of the substituted or unsubstituted amino groups represented by R₁ to R₁₄ include, but are not limited to, an amino group, an N-methylamino group, an N-ethylamino group, an N,N-dimethylamino group, an N,N-diethylamino group, an N-methyl-N-ethylamino group, an N-benzylamino group, an N-methyl-N-benzylamino group, an N,N-dibenzylamino group, an anilino group, an N,N-diphenylamino group, an N,N-dinaphthylamino group, an N,N-difluorenylamino group, an N-phenyl-N-tolylamino group, an N,N-ditolylamino group, an N-methyl-N-phenylamino group, an N,N-dianisolylamino group, an N-mesityl-N-phenylamino group, an N,N-dimesitylamino group, an N-phenyl-N-(4-tertiary butyl phenyl)amino group, and an N-phenyl-N-(4-trifluoromethylphenyl)amino group.

Examples of the aryl group represented by R₁ to R₁₄ include, but are not limited to, a phenyl group, a naphthyl group, an indenyl group, a biphenyl group, a terphenyl group, and a fluorenyl group.

Examples of the heterocyclic group represented by R₁ to R₁₄ include, but are not limited to, a pyridyl group, an oxazolyl group, an oxadiazolyl group, a thiazolyl group, a thiadiazolyl group, a carbazolyl group, an acridinyl group, and a phenanthrolyl group.

Examples of the substituents that may be contained in the alkyl group, the alkoxy group, the amino group, the aryl group, and the heterocyclic group include, but are not limited to, alkyl groups such as a methyl group, an ethyl group, a propyl group, and a tertiary butyl group, aralkyl groups such as a benzyl group, aryl groups such as a phenyl group and a biphenyl group, heterocyclic groups such as a pyridyl group and a pyrrolyl group, substituted amino groups such as a dimethylamino group, a diethylamino group, a dibenzylamino group, a diphenyl amino group, and a ditolylamino group, alkoxy groups such as a methoxyl group, an ethoxyl group, and a propoxyl group, aryloxy groups such as a phenoxyl group, halogen atoms such as fluorine, chlorine, bromine, and iodine atoms, and a cyano group.

In formula (1), R₁₁ or R₁₂ represents a substituted or unsubstituted fused polycyclic aromatic group. Examples of the fused polycyclic aromatic group represented by R₁₁ or R₁₂ include, but are not limited to, a naphthyl group, a fluoranthenyl group, a phenanthryl group, a benzofluoranthenyl group, a pyrenyl group, an anthryl group, a perylenyl group, a chrysenyl group, a naphthofluoranthenyl group, a fluorenyl group, a benzophenanthryl group, a quinolyl group, a carbazole group, an isoquinolyl group, a phenanthridyl group, and a phenanthrolyl group.

Examples of the substituents that may be contained in the fused polycyclic aromatic group include, but are not limited to, alkyl groups such as a methyl group, an ethyl group, a propyl group, and a tertiary butyl group, aralkyl groups such as a benzyl group, aryl groups such as a phenyl group, a 3,5-dimethylphenyl group, a 3,5-di-tert-butylphenyl group, and a biphenyl group, heterocyclic groups such as a pyridyl group and a pyrrolyl group, substituted amino groups such as a dimethylamino group, a diethylamino group, a dibenzylamino group, a diphenyl amino group, and a ditolylamino group, alkoxy groups such as a methoxyl group, an ethoxyl group, and a propoxyl group, aryloxy groups such as a phenoxyl group, halogen atoms such as fluorine, chlorine, bromine, and iodine atoms, and a cyano group.

The organic compound of this embodiment may be synthesized through the following synthetic route 1 by referring to NPL 1.

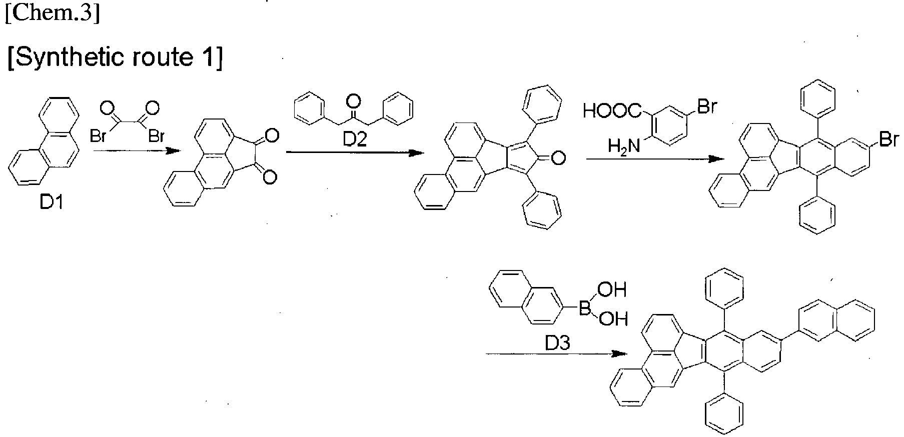

In particular, D1 (phenanthrene) is used as a starting material and reacted with BrC(=O)-C(=O)Br, D2 (acetone derivative) and a 2-aminobenzoic acid derivative containing a halogen atom substituting the 4-position or the 5-position to form a basic structure. The resulting product is reacted with D3 (boronic acid derivative) or a secondary-amino-containing heterocyclic compound to give a fused polycyclic aromatic group represented by R₁₁ or R₁₂. In the synthetic scheme described above, substituents may be introduced into positions of D1 other than the 1-position and the 10-position or the type of the substituent of D2 may be changed. As a result, substituents (alkyl groups, halogen atoms, phenyl groups, etc.) can be introduced into R₁ to R₁₀, R₁₃, and R₁₄ of the compound represented by formula (1). When a substituent is introduced into a fused polycyclic aromatic group forming the backbone of D3, the substituent is introduced into the fused polycyclic aromatic group represented by R₁₁ or R₁₂.

Synthetic examples of synthesizing the organic compound of this embodiment according to the synthetic route 1 are shown in Table 1.

**[Table 1]**

| | D1 | D2 | D3 | Synthesized compound |
|---|---|---|---|---|
| Synthetic example 1 | 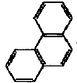 | 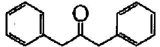 | 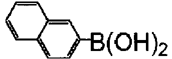 | 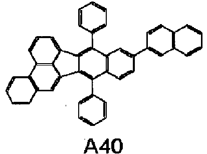 |
| Synthetic example 2 | 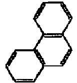 | 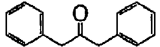 | 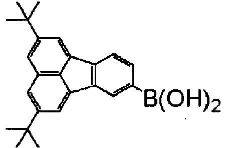 | 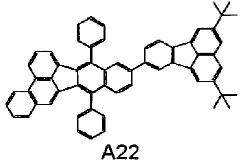 |
| Synthetic example 3 | 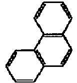 | 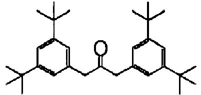 | 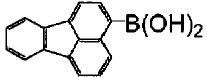 | 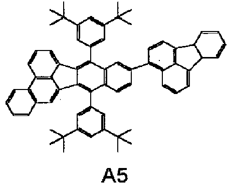 |
| Synthetic example 4 | 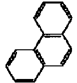 | 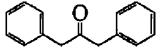 | 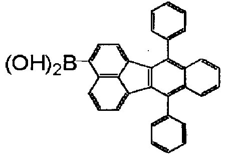 | 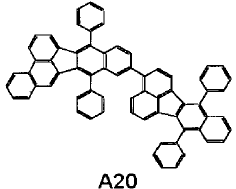 |
| Synthetic example 5 | 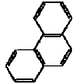 | 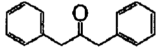 | 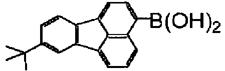 | 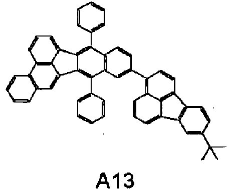 |
| Synthetic example 6 | 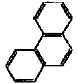 | 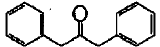 | 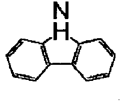 | 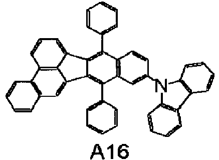 |

The organic compound of this embodiment will now be described in further detail.

Generally, in order to increase the emission efficiency of organic light-emitting devices, the emission quantum yield of the emission center material itself needs to be large. The inventors of the present invention have found through studies that the organic compound represented by general formula (1) exhibits a high quantum yield in a diluted solution. A high emission efficiency can be expected by using the organic compound as the constituent material of an organic light-emitting device.

The physical property of a light-emitting material suitable as a constituent material of an organic EL display, i.e., an image display apparatus, in particular, a material suitable as a blue light-emitting material, is that the emission peak of the emission material is at 430 nm to 480 nm. The organic compound of this embodiment has an emission peak at 430 nm to 480 nm, which is suitable for use in organic EL displays. Furthermore, the compound used as the light-emitting material of an organic light-emitting device is also required to have a basic structure that has a high quantum yield.

The basic structure of the organic compound of this embodiment is a dibenzo[b,k]fluoranthene backbone. The following backbones (benzo[k]fluoranthene backbone and benzo[b]fluoranthene backbone) are also available as similar basic structures having five-membered rings:

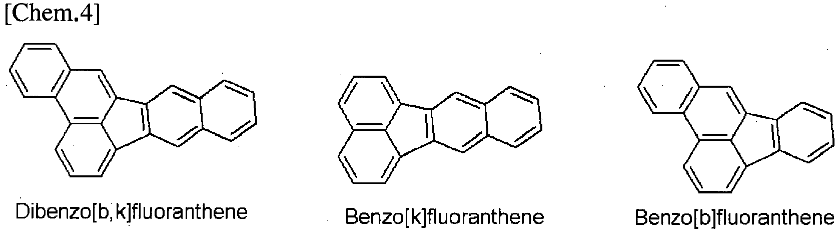

Quantum chemical calculation at the B3LYP/6-31G* level using a density functional theory shows that the oscillator strength of the basic structure of the organic compound of this embodiment is the highest, as shown in Table 2.

**[Table 2]**

| Compound | Absorption wavelength (S1) [nm] | Oscillator strength |
|---|---|---|
| Dibenzo[b,k]fluoranthene | 378 | 0.223 |
| Benzo[k]fluoranthene | 378 | 0.215 |
| Benzo[g]fluoranthene | 358 | 0.012 |

Since a high oscillator strength indicates a high quantum yield, the organic compound of this embodiment having a basic structure with a high quantum yield can be considered to be suitable for use as a light-emitting material.

However, as shown in Table 2, the dibenzo[b,k]fluoranthene (backbone) has a short absorption wavelength and the emission wavelength thereof is less than 430 nm. Thus, the basic structure of the organic compound, i.e., dibenzo[b,k]fluoranthene, by itself is not suitable as the light-emitting material since its emission wavelength is shorter than the blue emission wavelength. In order for a material to be used as a blue light-emitting material, the material needs to have an absorption wavelength peaking at about 390 nm considering the Stokes shift.

Because of this reason, the organic compound of this embodiment includes a substituent introduced into the 11-position or the 12-position of the basic structure shown below to effectively increase the wavelength.

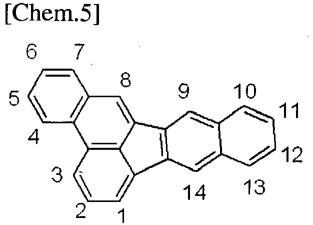

As a specific example, the case where a substituent is introduced into the 12-position is discussed. Note that when a substituent that contains many rotating portions is selected as the substituent, the quantum yield may decrease due to rotational vibrations. Thus, an aromatic ring substituent is desirably introduced to prevent the decrease in quantum yield due to rotational vibrations and to extend the absorption wavelength. The peaks of absorption wavelengths of the compounds shown below were measured and evaluated, the results of which are shown in Table 3.

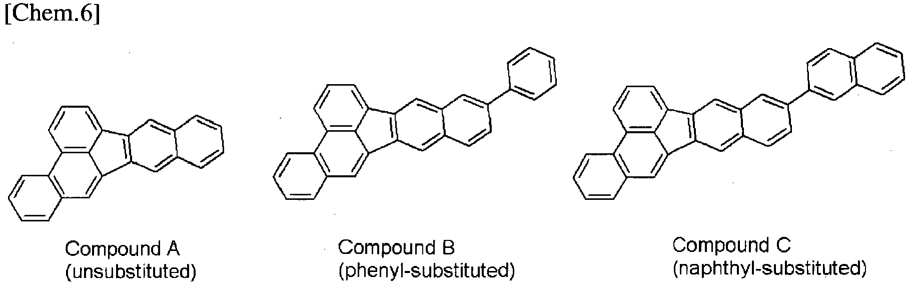

**[Table 3]**

| Compound | Absorption wavelength (S1) [nm] |
|---|---|
| Compound A (unsubstituted) | 376 |
| Compound B (phenyl-substituted) | 386 |
| Compound C (naphthyl-substituted) | 390 |

The results show that introduction of a naphthyl group, i.e., a substituent having a fused ring structure, can increase the peak absorption wavelength to about 390 nm. Thus, the substituent to be introduced into the 11-position or the 12-position is a fused ring aromatic group, which is a substituent having a fused ring structure. As a result, the organic compound represented by formula (1) becomes suitable as the blue light-emitting material.

Since the organic compound of this embodiment is highly planar, excimers are readily produced if no substituents are introduced. In order to suppress generation of excimers, substituents such as a phenyl group and an alkyl group can be introduced into the 9-position and the 14-position of the basic structure of the organic compound of this embodiment. In particular, when a phenyl group is introduced, the phenyl group is arranged orthogonal to the basic structure. This makes the structure of the entire molecule three-dimensional and suppresses stacking of the molecules. Accordingly, concentration quenching can be suppressed. Note that "orthogonal" means that the plane of the phenyl group is positioned orthogonal to the plane of the basic structure (dibenzo[b,k]fluoranthene).

No particular limitations are imposed on the type of substituents to be introduced into positions other than the 9-position, the 11-position, the 12-position and the 14-position. However, in order not to significantly change the physical properties of the basic structure, the substituents may contain hydrocarbons. In order to change the HOMO-LUMO of the compound, i.e., in order to change the emission color of the organic compound to color other than blue, such as green or red, a substituent containing a heteroatom can be introduced.

Specific examples of the organic compound of this embodiment are shown below.

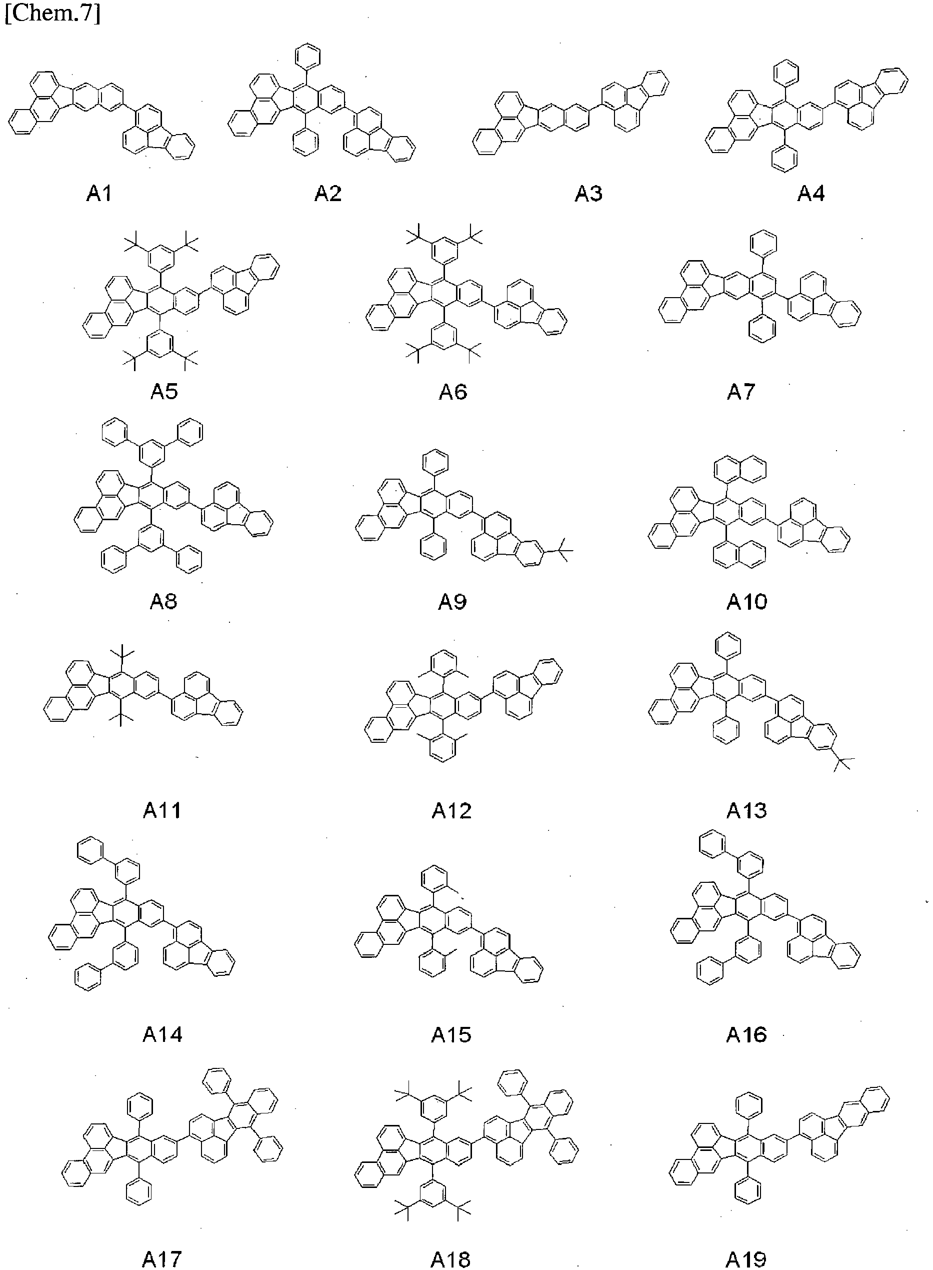

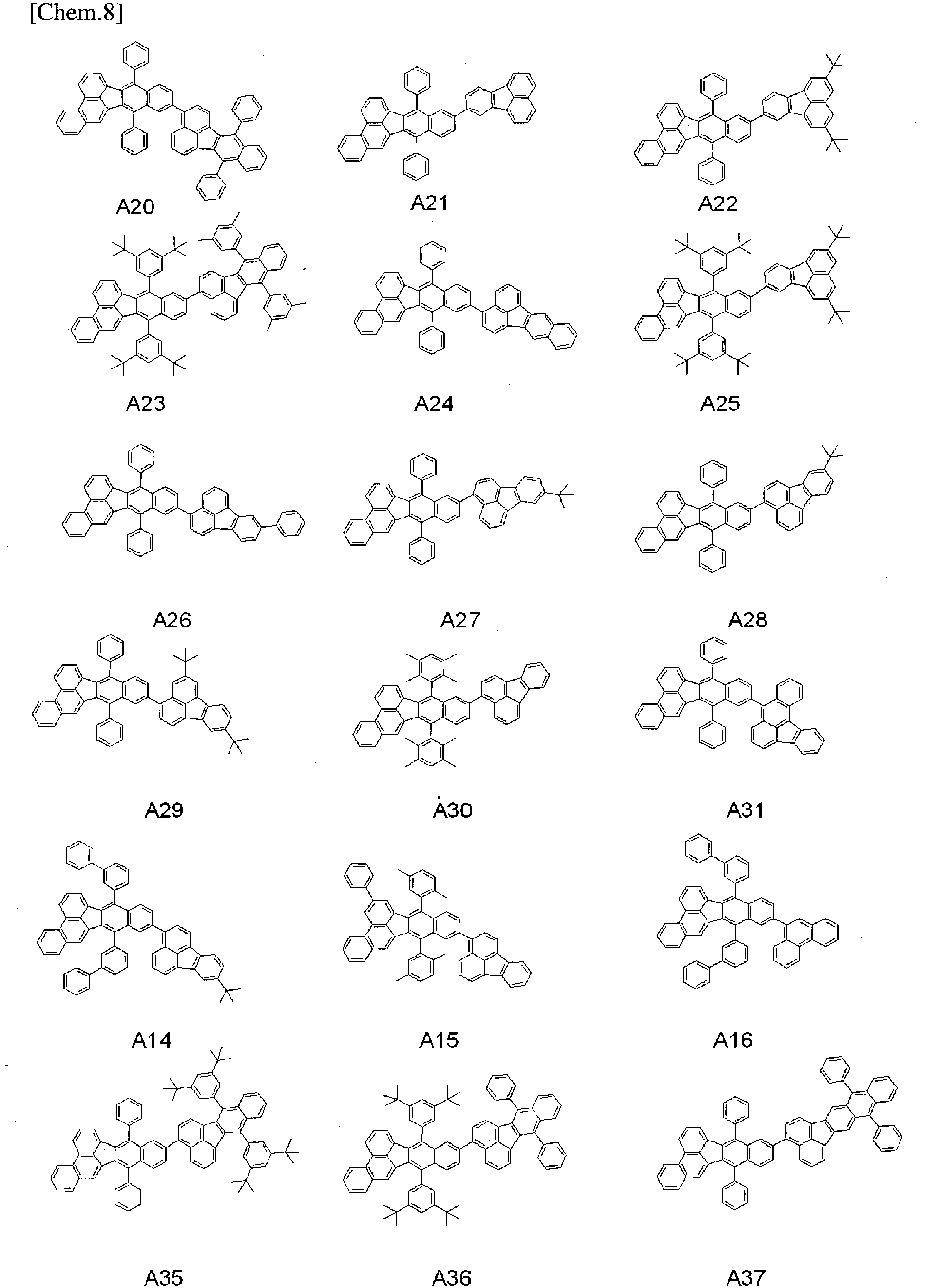

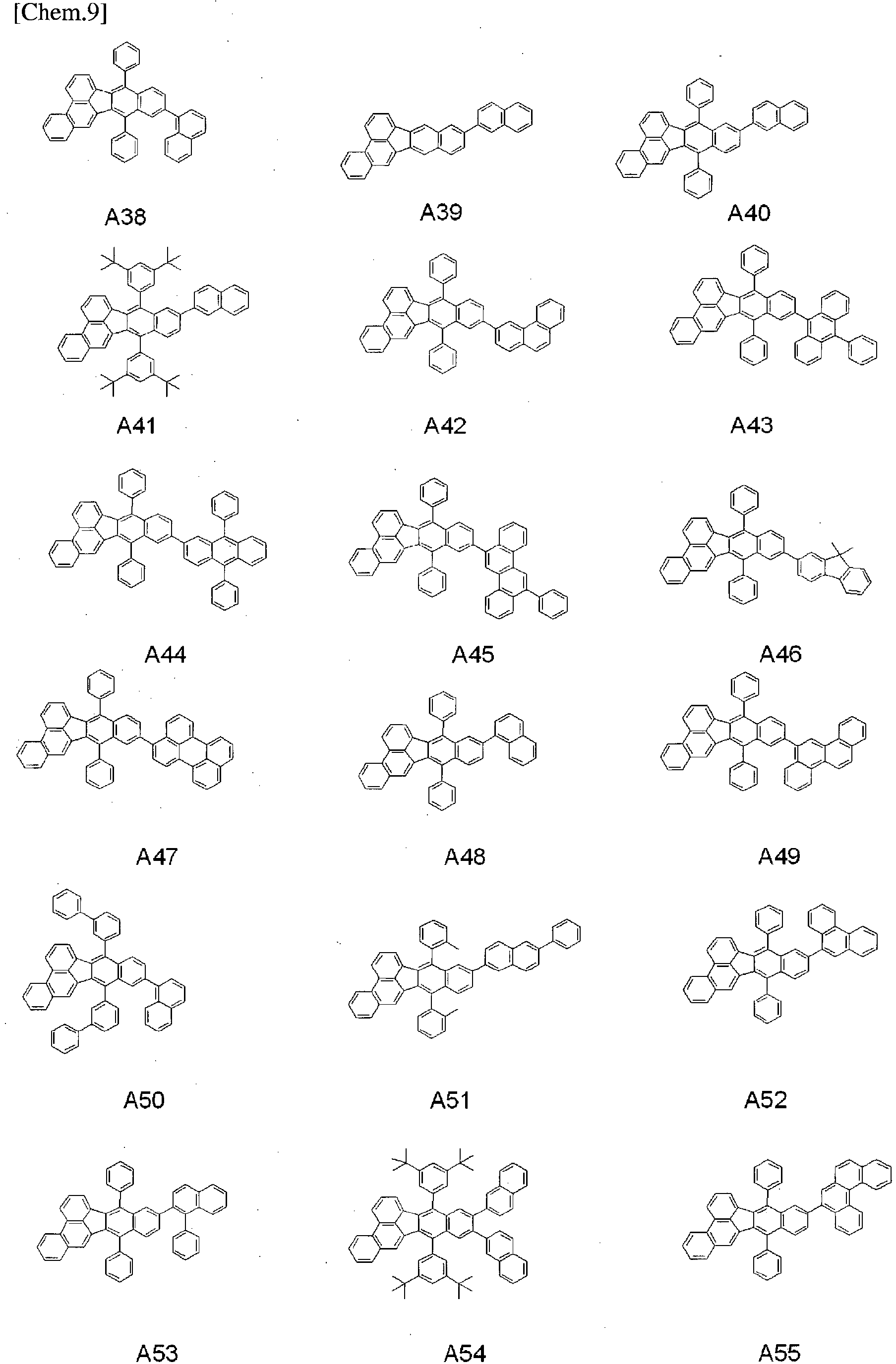

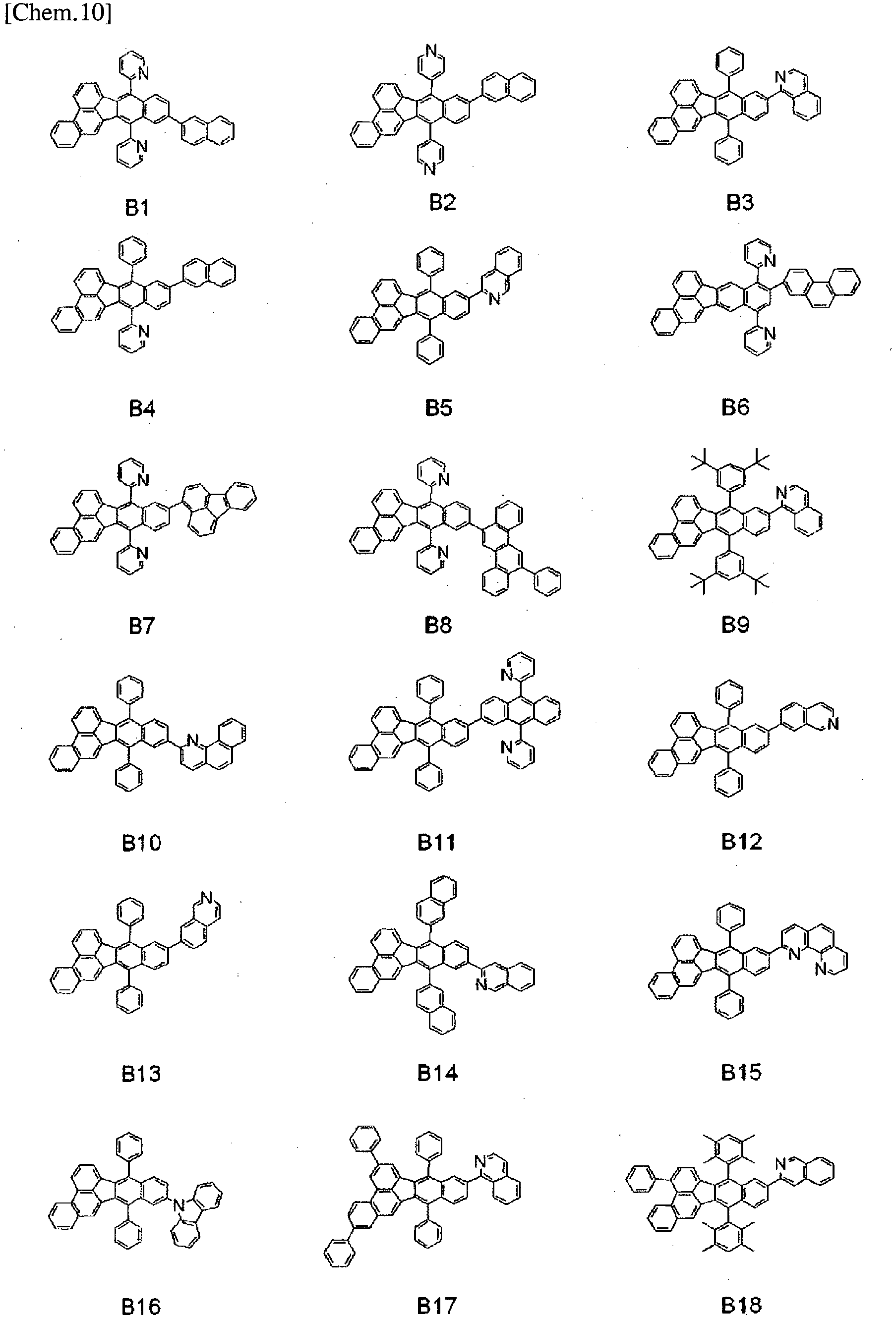

An organic light-emitting device according to another embodiment of the present invention will now be described.

The organic light-emitting device of this embodiment includes an anode, a cathode, and an organic compound layer interposed between the anode and the cathode. The organic compound layer of the organic light-emitting device contains the organic compound described above. The organic compound can be contained in an emission layer.

When the organic compound is contained in the emission layer, the emission layer may be composed of only the organic compound or may be constituted by a host and a guest.

When the emission layer is constituted by the host and the guest, the host is a material that has the largest weight ratio among the constituent materials of the emission layer, i.e., the material that serves as the main component. The guest is also referred to as "dopant" and is a material contained in the emission layer to serve as an auxiliary component together with an emission assist material, a charge injection material, etc. The organic compound may be used as the host or the guest. The organic compound is more suited to be used as the guest. When the organic compound is used as the guest, an organic light-emitting device that can output light at a high luminance and high efficiency and has significantly high durability can be obtained.

When the organic compound is used as the guest, the concentration of the guest relative to the host is preferably 0.01 wt% or more and 20 wt% or less and more preferably 0.5 wt% or more and 10 wt% or less.

Specific structural examples of the organic light-emitting device of this embodiment are described below. These specific examples are merely basic device configurations which do not limit the scope of the present invention.
(1) anode/emission layer/cathode
(2) anode/hole transport layer/electron transport layer/cathode
(3) anode/hole transport layer/emission layer/electron transport layer/cathode
(4) anode/hole injection layer/hole transport layer/emission layer/electron transport layer/cathode
(5) anode/hole transport layer/emission layer/hole-exciton blocking layer/electron transport layer/cathode

Various structures other than the structures of (1) to (5) may be employed. For example, an insulating layer, an adhesive layer, or an interference layer may be formed at the interface between an electrode and an organic compound layer. For example, an electron transport layer or a hole transport layer may be constituted by two layers having different ionization potentials.

If needed, the organic light-emitting device can use any other available compound in addition to the organic compound of the embodiment. In particular, the following compounds can be used.
(a) low-molecular-weight and high-molecular-weight hole injection compounds and hole transport compounds
(b) host compounds that serve as the host of the emission layer
(c) light-emitting compounds
(d) electron injection compounds and electron transport compounds

Examples of these compounds are described below.

The hole injection compound and the hole transport compound can be materials having high hole mobility. Examples of the low-molecular-weight and high-molecular-weight materials that have functions of injecting and transporting holes include, but are not limited to, triarylamine derivatives, phenylene diamine derivatives, stilbene derivatives, phthalocyanine derivatives, porphyrin derivatives, poly(vinylcarbazole), poly(thiophene), and other electrically conductive polymers.

Examples of the host compound include compounds shown in Table 4 below.
Derivatives of the compounds shown in Table 4 may also be used.

**[Table 4]**

| | | | |
|---|---|---|---|
| H1 | H2 | H3 | H4 |
| 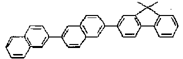 | 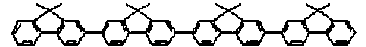 | 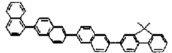 | 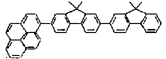 |
| H5 | H6 | H7 | H8 |
| 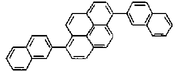 | 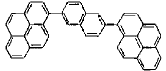 | 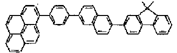 | 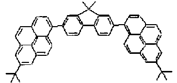 |
| H9 | H10 | H11 | H12 |
| 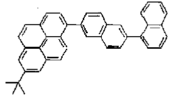 | 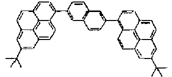 | 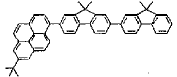 | 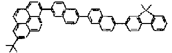 |
| H13 | H14 | H15 | H16 |
| 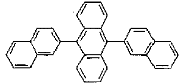 | 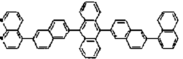 | 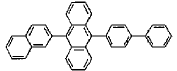 | 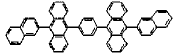 |
| H17 | H18 | H19 | H20 |
| 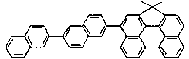 | 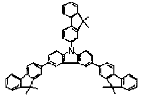 | 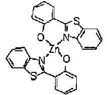 | 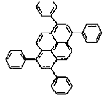 |
| H21 | H22 | H23 | H24 |
| 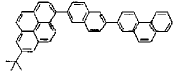 | 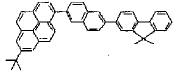 | 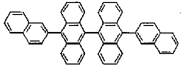 | 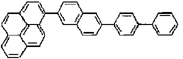 |
| H25 | H26 | H27 | H28 |
| 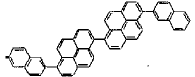 | 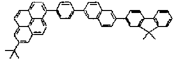 | 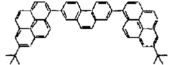 | 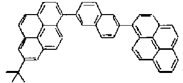 |

Other examples of the host compound include fused ring compounds (e.g., fluorene derivatives, naphthalene derivatives, anthracene derivatives, pyrene derivatives, carbazole derivatives, quinoxaline derivatives, and quinoline derivatives), organic aluminum complexes such as tris(8-quinolinolato)aluminum, organic zinc complexes, and polymer derivatives such as triphenylamine derivatives, poly(fluorene) derivatives, and poly(phenylene) derivatives. However, the present invention is not limited to these examples.

The electron injection compound and the electron transport compound are appropriately selected by considering, for example, the balance with the hole mobility of the hole injection compound and the hole transport compound. Examples of the compounds that have functions of injecting and transporting electrons include, but are not limited to, oxadiazole derivatives, oxazole derivatives, pyrazine derivatives, triazole derivatives, triazine derivatives, quinoline derivatives, quinoxaline derivatives, phenanthroline derivatives, and organic aluminum complexes.

The constituent material of the anode can have a large work function. Examples thereof include single metals such as gold, platinum, silver, copper, nickel, palladium, cobalt, selenium, vanadium, and tungsten, alloys of two or more of these single metals, and metal oxides such as tin oxide, zinc oxide, indium oxide, indium tin oxide (ITO), and indium zinc oxide. Electrically conductive polymers such as polyaniline, polypyrrole, and polythiophene can also be used. These electrode substances may be used alone or in combination. The anode may be constituted by single layer or two or more layers.

In contrast, the material of the cathode can have a small work function. Examples of the cathode material include single metals such as alkali metals, e.g., lithium, alkaline earth metals, e.g., calcium, aluminum, titanium, manganese, silver, lead, and chromium. Alloys of two or more of these single metals can also be used. For example, magnesium-silver, aluminum-lithium, and aluminum-magnesium can be used. Metal oxides such as indium tin oxide (ITO) can also be used. These electrode substances may be used alone or in combination. The cathode may be constituted by single layer or two or more layers.

In the organic light-emitting device according to this embodiment, a layer that contains the organic compound of this embodiment and layers composed of other organic compounds are formed by the following method. Typically, thin films are formed by vacuum vapor deposition, ionized evaporation, sputtering, plasma, or a coating technique in which a material is dissolved in an appropriate solvent (e.g., spin-coating, dipping, casting, a Langmuir-Blodgett technique, and an ink jet technique). When layers are formed by vacuum deposition or a solution coating technique, crystallization does not readily occur and stability overtime is improved. When a coating technique is used to form films, an appropriate binder resin may be used in combination to form films.

Examples of the binder resin include, but are not limited to, polyvinyl carbazole resins, polycarbonate resins, polyester resins, ABS resins, acrylic resins, polyimide resins, phenol resins, epoxy resins, silicone resins, and urea resins. These binder resins may be used alone as a homopolymer or in combination as a copolymer. If necessary, additives such as plasticizers, antioxidants, and UV absorbers may be used together.

The organic light-emitting device of this embodiment can be used in display apparatuses and lighting apparatuses. The organic light-emitting device can also be used as the exposure light source of an electrophotographic image-forming apparatus or a backlight of a liquid crystal display apparatus.

When the organic light-emitting device of this embodiment is used as a component of a display apparatus, the organic light-emitting device is installed in a display unit. The display unit includes a plurality of pixels and the organic light-emitting device functions as a pixel. The display apparatus also includes a unit that supplies electrical signals to the organic light-emitting device. The display apparatus can also be used as an image display apparatus of a personal computer or the like.

The display apparatus may be used in a display unit of an imaging apparatus such as a digital camera and a digital video camera. An imaging apparatus is an apparatus that includes a display unit and an imaging unit that includes an imaging optical system for capturing images.

An image display apparatus equipped with the organic light-emitting device of this embodiment will now be described.

Fig. 1 is a schematic cross-sectional view showing an example of an image display apparatus equipped with the organic light-emitting device of this embodiment.

An image display apparatus 1 shown in Fig. 1 includes a substrate 11 such as a glass substrate and a moisture-proof film 12 on the substrate 11. The moisture-proof film 12 protects a TFT or organic compound layers. A gate electrode 13 composed of chromium or the like is formed on the moisture-proof film 12. A gate insulating film 14 is formed over the gate electrode 13. A semiconductor layer 15 is formed over the gate insulating film 14.

A TFT element 18 includes the semiconductor layer 15, a drain electrode 16, and a source electrode 17. An insulating film 19 is provided on the top of the TFT element 18. The source electrode 17 is connected to an anode 111 of the organic light-emitting device through a contact hole (through hole) 110.

Although an organic compound layer 112 is illustrated as a single layer shown in Fig. 1, the organic compound layer 112 is actually a laminate constituted by two or more layers. In order to suppress deterioration of the organic light-emitting device, a first protective layer 114 and a second protective layer 115 are formed on a cathode 113.

The luminance of the emission from the organic light-emitting device is controlled by electric signals supplied from the TFT element 18. Since plural light-emitting devices are provided on the surface, an image can be displayed by controlling the emission luminance of the respective light-emitting devices.

When a display apparatus using the organic light-emitting devices of the embodiment is driven, high-quality images can be stably displayed over a long time.

### Examples

The present invention will now be described in further detail by using Examples below.

### Example 1

### Synthesis of example compound A4

### (1) Synthesis of intermediate E5

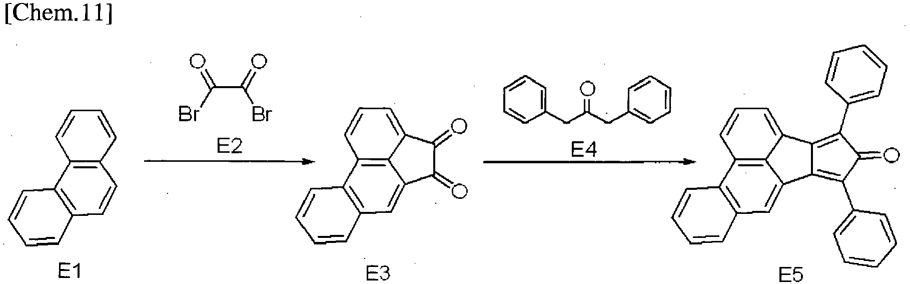

### (1-1) Synthesis of intermediate E3

Reagents and solvents described below were charged into a reactor:
phenanthrene (E1): 17.8 g (100 mmol)
E2: 21.5 g (100 mmol)
aluminum bromide: 26.6 g (100 mol)
carbon disulfide: 500 ml

After the reaction solution was cooled to -40 degrees Celsius, stirring was conducted for 3 hours at this temperature (-40 degrees Celsius). After the reaction solution was warmed to room temperature, stirring was conducted for 1 hour at this temperature (room temperature). The reaction solution was discharged into water and precipitated solids were filtered, washed with ethanol, and dried to obtain 20 g (yield: 85%) of E3 in form of ocher solids.

### (1-2) Synthesis of intermediate E5

Reagents and solvents described below were charged into a reactor:
E3: 11.6 g (50 mmol)
E4: 10.5 g (50 mmol)
ethanol: 200 ml

The reaction solution was heated to 60 degrees Celsius and a 5 M aqueous sodium hydroxide solution (20 ml) was added thereto dropwise. Upon completion of addition, the reaction solution was heated to 80 degrees Celsius and stirring was conducted for 2 hours at this temperature (80 degrees Celsius). Upon completion of the reaction, the reaction solution was cooled and precipitated solids were filtered and washed with water and then ethanol. Next, vacuum thermal drying was conducted at 80 degrees Celsius to obtain 18.2 g (yield: 95%) of E5 in form of dark green solids.

### (2) Synthesis of example compound A4

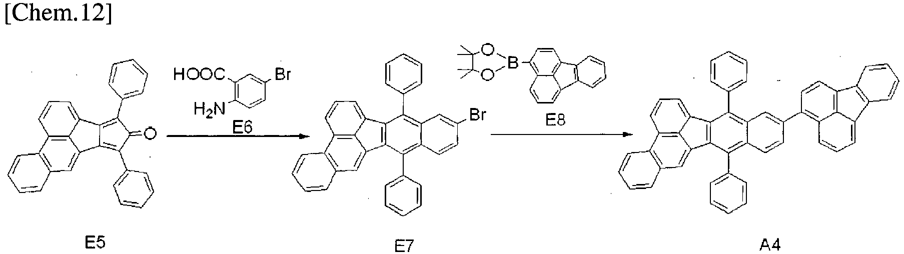

### (2-1) Synthesis of intermediate E7

Reagents and solvents described below were charged into a reactor:
E5: 4.1 g (10 mmol)
E6: 2.7 g (11 mmol)
toluene: 100 ml

The reaction solution was heated to 80 degrees Celsius and isoamyl nitrite (1.3 g, 11 mmol) was slowly added thereto dropwise. Next, the reaction solution was heated to 110 degrees Celsius and stirring was conducted for 3 hours at this temperature (110 degrees Celsius). Upon completion of the reaction, the reaction solution was cooled, washed with 100 ml of water twice, washed with saturated saline, and dried with magnesium sulfate. The reaction solution was filtered and the filtrate was condensed under vacuum to obtain a brown liquid. The liquid was purified by column chromatography (developing solvent: toluene/heptane = 1/1), and recrystallized with chloroform/methanol to obtain 4.37 g (yield: 82%) of E7 in form of crystals.

### (2-2) Synthesis of example compound A4

Reagents and solvents described below were charged into a 100 ml round-bottomed flask:
E7: 1067 mg (2 mmol)
E8: 656 mg (2 mmol)
Pd(PPh₃)₄: 0.05 g
toluene: 20 ml
ethanol: 10 ml
2M aqueous sodium carbonate solution: 20 ml

The reaction solution was stirred for 8 hours at 80 degrees Celsius under a nitrogen stream. Upon completion of the reaction, the precipitated crystals were isolated by filtering and sequentially washed with water, ethanol, and heptane. The crystals were dissolved in hot toluene and the resulting solution was subjected to hot filtration. The solution was then recrystallized with toluene/heptane. The crystals obtained by the recrystallization were vacuum dried at 120 degrees Celsius and purified by sublimation to obtain 968 mg (yield: 74%) of example compound A4 in form of pale yellow crystals.

The emission spectrum of the obtained example compound A4 in toluene solution (1 X 10⁻⁵ mol/l) was measured. In particular, photoluminescence at an excitation wavelength of 350 nm was measured with F-4500 produced by Hitachi Ltd. As a result, an emission spectrum having an emission peak (maximum intensity) at 438 nm was obtained.

### Example 2

### Synthesis of example compound A27

Example compound A27 (910 mg) was obtained by synthesis as in Example 1 except that E8 of Example 1, (2-2) was changed to E9 (767 mg, 2 mmol) represented by the formula below:

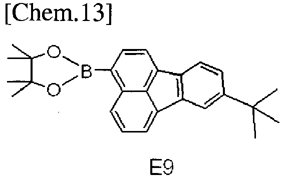

The emission spectrum of the obtained example compound A27 was also measured as in Example 1. As a result, an emission spectrum having an emission peak (maximum intensity) at 440 nm was obtained.

### Example 3

An organic light-emitting device in which an anode, a hole injection layer, a hole transport layer, an emission layer, a hole/exciton blocking layer, an electron transport layer, and a cathode were sequentially stacked in that order on a substrate was prepared by the following method.

First, an ITO film was formed on a glass substrate. The thickness of the ITO film was 100 nm. The ITO film was patterned into a desired shape. The patterned ITO film functions as an anode.

Then organic compound layers (hole injection layer/hole transport layer/emission layer/hole-exciton blocking layer/electron transport layer) and a cathode were sequentially formed on the anode. These films were continuously formed by vacuum vapor deposition by resistance heating in a vacuum chamber of 10⁻⁵ Pa so that the opposing electrode area was 3 mm². Table 5 shows the constituent materials and thickness of the layers constituting the light-emitting device of this example.

**[Table 5]**

| | | Constituent material | Thickness |
|---|---|---|---|
| Hole transport layer | | G-1 | 30 nm |
| Emission layer | | G-2 (host) Example compound A2 (guest) (host:guest = 95:5 (weight ratio)) | 30 nm |
| Hole-exciton blocking layer | | G-3 | 10 nm |
| Electron transport layer | | G-4 | 30 nm |
| Cathode | First metal electrode layer | LiF | 1 nm |
| | Second metal electrode layer | Al | 100 nm |

Structural formulae of compounds G-1 to G-4 in Table 5 are as follows.

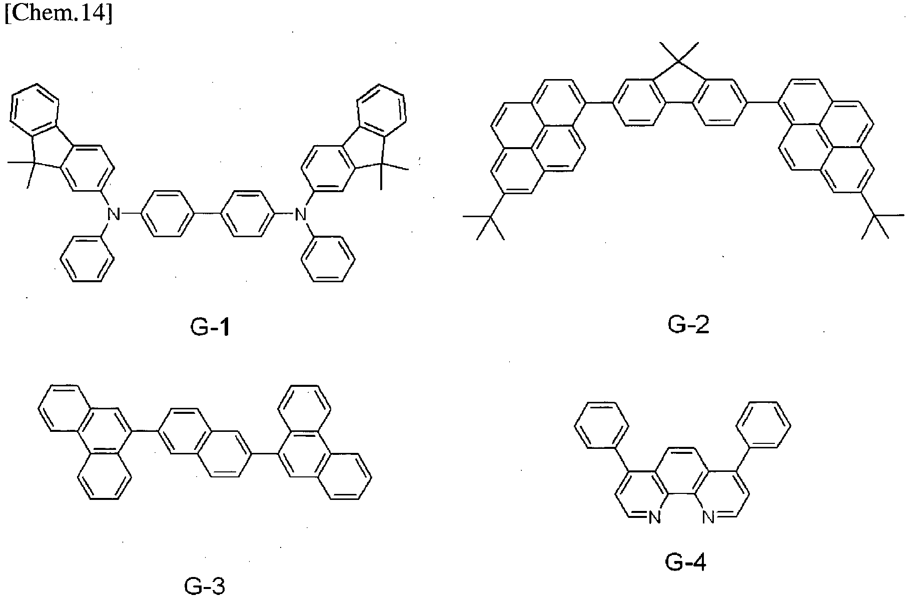

The properties of the resulting organic light-emitting devices were evaluated. In particular, the current-voltage characteristic was measured with a microammeter 4140B produced by Hewlett-Packard Co., and the emission luminance was measured with BM7 produced by TOPTON CORPORATION. The driving voltage was 4.5 V and the emission efficiency was 3.5 cd/A.

### EXAMPLES 4 TO 9

The combination of the host and the guest in Example 3 was changed to those indicated in Table 6 below. Organic light-emitting devices were prepared as in Example 3 except for these combinations. The organic light-emitting devices were evaluated as in Example 3. The results are shown in Table 6.

**[Table 6]**

| | Host | Guest | Driving voltage (V) | Emission efficiency (cd/A) |
|---|---|---|---|---|
| Example 4 | H23 | A4 | 4.8 | 3.6 |
| Example 5 | H11 | A4 | 4.5 | 3.4 |
| Example 6 | H22 | A17 | 5.0 | 3.8 |
| Example 7 | H22 | A22 | 5.2 | 3.2 |
| Example 8 | H17 | A27 | 5.6 | 3.6 |
| Example 9 | H10 | B16 | 6.0 | 2.2 |

As described above, the organic compounds according to embodiments of the present invention exhibit high quantum yields and high emission characteristics. Thus, when the organic compounds are used as the constituent materials of organic light-emitting devices, the organic light-emitting devices can achieved good emission characteristics, namely, high efficiency and high luminance. Furthermore, according to embodiments of the present invention, a organic compound that is suitable for a constituent material of a blue light-emitting device can be provided.

This application claims the benefit of Japanese Patent Application No. 2009-185556, filed August 10, 2009.

## Claims

1. An organic compound represented by general formula (1) 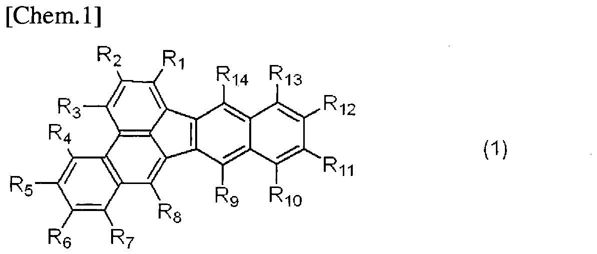 (where R₁ to R₁₄ each independently represent a hydrogen atom, a halogen atom, a substituted or unsubstituted alkyl group, a substituted or unsubstituted alkoxy group, a substituted or unsubstituted amino group, a substituted or unsubstituted aryl group, or a substituted or unsubstituted heterocyclic group, and R₁₁ or R₁₂ represents a substituted or unsubstituted fused polycyclic aromatic group.)

2. An organic light-emitting device comprising:
an anode;
a cathode; and
an organic compound layer interposed between the anode and the cathode,
wherein the organic compound layer contains the organic compound according to Claim 1.

3. The organic light-emitting device according to Claim 2, wherein the organic compound layer containing the organic compound is an emission layer.

4. An image display apparatus comprising:
a plurality of pixels each including the organic light-emitting device according to Claim 2; and
a unit configured to supply electrical signals to the organic light-emitting device.

## Patentansprüche

1. Organische Verbindung, die durch die allgemeine Formel (1) dargestellt ist 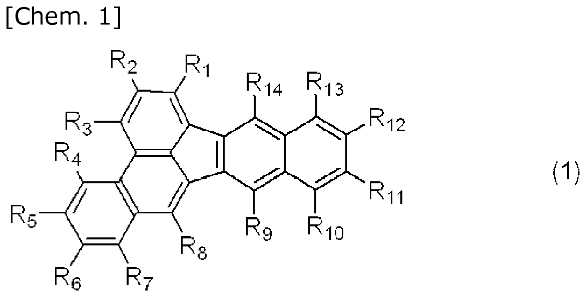 (wobei R₁ bis R₁₄ jeweils unabhängig ein Wasserstoffatom, ein Halogenatom, eine substituierte oder unsubstituierte Alkylgruppe, eine substituierte oder unsubstituierte Alkoxygruppe, eine substituierte oder unsubstituierte Aminogruppe, eine substituierte oder unsubstituierte Arylgruppe oder eine substituierte oder unsubstituierte heterozyklische Gruppe darstellen und R₁₁ oder R₁₂ eine substituierte oder unsubstituierte kondensierte, polycyclische, aromatische Gruppe darstellt.)

2. Organische Licht-emittierende Vorrichtung, die umfasst:
eine Anode;
eine Kathode; und
eine organische-Verbindung-Schicht, die zwischen der Anode und der Kathode eingefügt ist,
wobei die organische-Verbindung-Schicht die organische Verbindung nach Anspruch 1 enthält.

3. Organische Licht-emittierende Vorrichtung nach Anspruch 2, wobei die organische-Verbindung-Schicht, die die organische Verbindung enthält, eine Emissionsschicht ist.

4. Bilddisplaygerät, die umfasst:
eine Mehrzahl an Pixeln, die jeweils die organische Licht-emittierende Vorrichtung nach Anspruch 2 beinhalten; und
eine Einheit, die zum Zuführen elektrischer Signale zu der organischen Licht-emittierenden Vorrichtung konfiguriert ist.

## Revendications

1. Composé organique représenté par la formule générale (1) 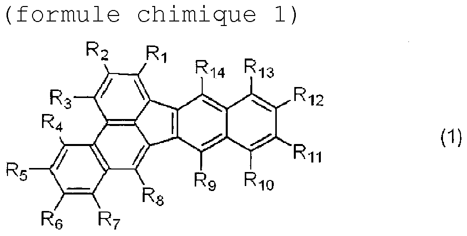 dans laquelle R₁ à R₁₄ représentent chacun indépendamment un atome d'hydrogène, un atome d'halogène, un groupe alkyle substitué ou non substitué, un groupe alkoxy substitué ou non substitué, un groupe amino substitué ou non substitué, un groupe aryle substitué ou non substitué, ou un groupe hétérocyclique substitué ou non substitué, et R₁₁ ou R₁₂ représente un groupe aromatique polycyclique condensé substitué ou non substitué.

2. Dispositif luminescent organique comprenant :
une anode ;
une cathode ; et
une couche de composé organique intercalée entre l'anode et la cathode,
dans lequel la couche de composé organique contient le composé organique selon la revendication 1.

3. Dispositif luminescent organique selon la revendication 2, dans lequel la couche de composé organique contenant le composé organique est une couche d'émission.

4. Appareil d'affichage d'images comprenant :
une pluralité de pixels, chacun comprenant le dispositif luminescent organique selon la revendication 2 ; et
une unité configurée de manière à fournir des signaux électriques au dispositif luminescent organique.
